# EUROPEAN PATENT SPECIFICATION

(11) **EP 3 870 227 B1**
(45) Date of publication and mention of the grant of the patent: **21.12.2022**
(21) Application number: 19828892.0
(22) Date of filing: 23.10.2019
(51) Int. Cl.: A61K 47/10, A61K 47/12, A61K 47/14, A61K 9/08

(54) **DEEP EUTECTIC SOLVENT PLATFORM FOR ORAL PHARMACEUTICAL FORMULATIONS**
TIEFEUTEKTISCHE LÖSUNGSMITTELPLATTFORM FÜR ORALE PHARMAZEUTISCHE FORMULIERUNGEN
PLATEFORME DE SOLVANT EUTECTIQUE PROFOND POUR FORMULATIONS PHARMACEUTIQUES ORALES

(30) Priority: 24.10.2018 NL 2021864; 26.03.2019 NL 2022813; 05.04.2019 NL 2022884
(43) Date of publication of application: 01.09.2021
(73) Proprietor: SeraNovo B.V., 2333 CH Leiden (NL)
(72) Inventor: KLUFT, Bastiaan, 2333 CH Leiden (NL); HODGINS, Niall, 2333 CH Leiden (NL)
(74) Representative: V.O.
(86) International application number: PCT/NL2019/050697
(87) International publication number: WO 2020/085904

(56) References cited:
- WO-A1-97/00670
- US-A1- 2012 014 893
- US-A1- 2012 232 152

## Description

The present invention relates to Eutectic Mixtures (EM), and preferably Deep Eutectic Solvents (DES), that are used in or as oral pharmaceutical formulation platforms. These platforms improve the solubility and bioavailability of active pharmaceutical ingredients (API), and especially of poorly water-soluble APIs. Liquid formulations are accepted to hold benefits over advanced solid formulation technologies, such as solid dispersions. For example, liquids formulations offer dose flexibility, are often faster to develop and have simpler scale up processes.

Particularly, the present invention is directed to a platform used to create liquid formulations and especially non-aqueous liquid formulations. That is, a liquid formulation platform aimed at formulating poorly water soluble active pharmaceutical ingredients. For a particular active ingredient, a liquid formulation may be designed, for which the designed liquid formulation is to be administered orally and should increase the bioavailability of the active ingredient.

DESs are liquids with a melting point that is lower than the melting points of its constituents. The present invention uses deep eutectic solvents that are liquid at ambient conditions (room temperature (25 °C); 1 atm.) and have negligible human or animal toxicity in the used doses. A NADES is a DES composed of naturally occurring or nature derived constituents. When used in pharmaceutical compositions, the DESs should not be unacceptably toxic to a human or animal to be treated therewith.

As the skilled person knows, poorly water-soluble APIs require advanced formulation technology in order to be effectively absorbed in the gastrointestinal (GI) tract when orally administered. Without the APIs dissolving in aqueous solutions at systemic pH ranges, the absorption of APIs will be very variable and poor which limits the therapeutic effects of the APIs. An "aqueous environment" as employed herein generally means a gastrointestinal fluid if *in vivo* and an aqueous test medium if *in vitro.* More specifically, "aqueous environment" encompasses, if the aqueous environment is *in vivo* and has a pH in the range of 1.0 to 2.0, the stomach; and if the aqueous environment is *in vivo* and has a pH in the range of 5.0 to 8.0, the intestine.

When creating liquid formulations for use as pharmaceutical compositions there are a number of problems to overcome, of which the most relevant ones are discussed herein-below.

Firstly, a sufficiently large concentration of an API or drug (which term will be used as synonym for API herein-below) must be reached in the liquid such that an administrable volume should contain a sufficient amount of drug for an effective dose. For example, a 100 mg/ml concentration to deliver a 100 mg dose in 1 ml is generally an acceptable volume, whereas a 1 mg/ml concentration requiring 100 ml for the same dose generally is unacceptable.

Further, the API must be stable over an acceptable period of time in the liquid formulation. An acceptable period of time in this respect may be interpreted as an acceptable shelf-life of the liquid formulation, depending on the specific API incorporated therein.

Further, the API when administered orally must be in a form that can be absorbed by/in the GI tract. For example, the API must be solubilized in the in *vivo* aqueous environment.

When these problems are solved or do not occur, a suitable dose of API can be orally administered and absorbed by a patient, thus creating an effective formulation.

In WO 97/00670, biologically active compositions are described to increase the solubility of active ingredients at the time. However, the drawback of these composition is that the bioavailability in the gastrointestinal (GI) tract is not improved because the composition are not, or only very poorly, capable of dissolving polymeric precipitation inhibitors. Moreover the composition described are not suitable for oral administration. These compositions therefore do not solve the above-mentioned problems.

In US 2012/0232152 and US 2012/0014893 topical formulations based on liquid solvents, e.g. ethanol and acetone are disclosed. These formulations are no DES and are not suitable for oral administration. These compositions therefore do not solve the above-mentioned problems.

The present invention provides systems to prepare pharmaceutical compositions which do not have, and hence solve the problems mentioned herein-above, or at least considerably reduce such problems. A full active dose of API can be dissolved in the designed liquid. Further, the API is stable in the formulation over time. Further, the API is sufficiently soluble in the *in* vivo aqueous environment to be absorbed.

In a first aspect, the invention relates to a deep eutectic solvent (DES) composition, comprising a combination of a glycol with a polymer solubilizer component, which polymer solubilizer component is selected from the group consisting of esters and lactones of organic acids; dicarboxylic acids; esters of dicarboxylic acids; esters, ethers and carbonates of diols and triols; and mixtures thereof, in a molar ratio of a glycol to the polymer solubilizer component in the range of between 12 to 1 and 1 to 10, preferably in the range of 12 to 1 and 1 to 4, more preferably in a range of between 12 to 1 to 1:2, even more preferably in the range of between 8 to 1 and 1 to 2, even more preferably in the range of between 8 to 1 and 1 to 1.5 and most preferably in a range of between 4 to 1 and 1 to 1; the composition further comprising at least one DES constituent.

In terms of absolute moles, the molar ratio of the polymer solubilizer component to a glycol may be translated to a formulation wherein the amount of a glycol is used in a range between 0.1 to 3 moles, preferably in the range of 0.5 to 3 moles, more preferably in a range of 1 to 2 moles and wherein, accordingly, the polymer solubilizer component is used in a range between 0.1 to 2 moles, preferably in the range between 0.25 to 2 moles, and more preferably in a range of 0.5 to 1.5 moles.

Increasing the amount of polymer solubilizer allows the dissolution of increased amounts of polymeric precipitation inhibitor (PPI) in the DES, as described herein-below.

The core of the present invention is hence a mixture of a glycol (G) and the specific polymer solubilizer in the indicated ratio together with at least one DES, preferably NADES, constituent. Or in other words, a DES composition comprising a glycol (G) and the specific polymer solubilizer in the indicated ratio together with at least one DES constituent, wherein the DES constituent preferably comprises at least one NADES constituent. The presence of the at least one DES or NADES constituent - described in further detail herein-below - in the DES system according to the invention serves to optimize the solvent for a given API and/or to increase the solubility of the API. Sometimes, better results are obtained when a combination of two or more additional DES or NADES constituents are included. The molar ratio of at least one DES constituent, preferably NADES constituent(s) to the selected glycol or polymer solubilizer constituent lies in the range between 0.1 to 1 and 4 to 1 for each constituent, preferably in the range between 0.2 to 1 and 2 to 1 and more preferably in the range between 0.3 to 1 and 1 to 1. An exemplary DES system formulated using a glycol, polymer solubilizer constituent, and NADES constituent in a molar composition of the selected glycol to polymer solubilizer constituent to NADES constituent of 2 to 0.5 to 1, would accordingly have a molar ratio of NADES constituent to the selected or polymer solubilizer of 2. An exemplary DES system formulated using a glycol, polymer solubilizer constituent, and first and second NADES constituent (NADES1, NADES2) in a molar composition of a glycol to polymer solubilizer to NADES1 constituent to NADES2 constituent of 2 to 0.5 to 1 to 1, would accordingly have a ratio of NADES1 constituent to a polymer solubilizer of 2 and NADES2 constituent to a polymer solubilizer of 2.

When an API or drug is added to the DES of the present invention, a pharmaceutical composition is obtained. It goes without saying that the concentration of an API in the DES according to the invention is dependent on the maximum solubility of the API within the designed liquid, the desired concentration in the formulation and the effect of concentration on the bioavailability.

The term "active pharmaceutical ingredient" (API) can be used interchangeably with the terms "drug", "(bio-)active compound", "therapeutic agent", *etc.*

The pharmaceutical compositions of the present invention are based on the liquid DES formulations prepared in order to improve the solubility/ bioavailability of an API. That is, the invention combines a DES composed of a glycol, a polymer solubilizer, and one or more low toxicity (NA)DES constituents (optionally together with other ingredients or constituents, such as one or more pharmaceutically acceptable, biocompatible Polymeric Precipitation Inhibitors (PPIs) as described in more detail herein-below).

As mentioned herein-above, the invention intends to deal with poorly water soluble active pharmaceutical ingredients (API). The API may for example have an aqueous solubility of not more than 1 mg/mL at pH 6.8 when it is a weakly basic compound, of no more than 1 mg/mL at pH 1.2 when it is a weakly acidic compound, and of no more than 1 mg/mL at any pH between the physiological pH of 1.0-8.0 for neutral or non-ionisable compounds. The solubility of an API can be determined by adding the highest dose strength in 250 mL of aqueous solutions with a pH ranging from 1 to 7.4 to cover GI physiological conditions. If the highest dose strength of API is not dissolved in 250 mL of solution of any pH from 1-7.4, the API is considered to be poorly water soluble.

As used herein, the term "weakly basic compound", as well as reference to any specific new chemical entity, drug, or active pharmaceutical ingredient, includes the base, pharmaceutically acceptable salts, polymorphs, stereoisomers, solvates, esters and mixtures thereof, which is a chemical base in which protonation is incomplete in an aqueous medium. In one embodiment, the weakly basic compound of the compositions of the present invention can refer to a compound having at least one pKa in the range of less than 14, wherein pKa can be measured or by calculation. In another embodiment, the weakly basic compound of the compositions of the present invention can refer to a compound having at least one pKa of less than 14, which has a pH dependent solubility between physiological pH with a lower solubility at higher pH. In another embodiment, the weakly basic drug of the compositions of the present invention can refer to a compound having at least one pKa of 0.0-10.0, which has a pH dependent solubility between physiological pH of 1.0-8.0 with a lowest solubility at around pH 6.0-8.0. In another embodiment, the weakly basic compound has a solubility of not more than about 1 mg/mL at pH 6.8. In another embodiment, the weakly basic compound includes at least one basic nitrogen atom. In yet another embodiment, the weakly basic compound has a pKa of less than 14, and a solubility of not more than about 1 mg/mL at pH 6.8. In yet another embodiment, the weakly basic compound has a pKa of less than 14, and includes at least one basic nitrogen atom. In yet another embodiment, the weakly basic compound as a pKa of less than 14, a solubility of not more than 1 mg/mL at pH 6.8, and includes a least one basic nitrogen atom.

As used herein, the term "weakly acidic compound" as well as reference to any specific new chemical entity, drug, or active pharmaceutical ingredient, includes the acid, pharmaceutically acceptable salts, polymorphs, stereoisomers, solvates, esters and mixtures thereof, which is a chemical base in which deprotonation is incomplete in aqueous medium. In one embodiment, the weakly acidic drug of the compositions of the present invention can refer to a compound having at least one pKa of less than 14, wherein pKa can be measured or by calculation. In another embodiment, the weakly acidic compound of the compositions of the present invention can refer to a compound having at least one pKa of less than 14, which has a pH dependent solubility between physiological pH with a lower solubility at lower pH. In another embodiment, the weakly acidic drug of the compositions of the present invention can refer to a compound having at least one pKa of 0.0-10.0, which has a pH dependent solubility between physiological pH of 1.0-8.0 with a lower solubility around pH 1.0-2.0. In another embodiment, the weakly acid compound has a solubility of not more than about 1 mg/mL at pH 1.0-2.0. In another embodiment, the weakly acidic compound includes at least one acidic functional group. In yet another embodiment, the weakly acidic compound has at least one pKa of less than 14, and a solubility of not more than about 1 mg/mL at pH 1.2. In yet another embodiment, the weakly acidic compound has a pKa of less than 14, and includes at least one acidic functional group. In yet another embodiment, the weakly acidic compound has a pKa of less than 14, a solubility of not more than 1 mg/mL at pH 1.2, and includes a least one acidic functional group.

The term "neutral or non-ionizable compound" as well as reference to any specific new chemical entity, drug, or active pharmaceutical ingredient, includes polymorphs, stereoisomers, solvates, esters and mixtures thereof. The neutral or non-ionizable API of the compositions of the present invention can refer to a compound that has a neutral form or does not have an ionizable functional group in the pH range of below 14. In one embodiment, the neutral or non-ionizable compound has a pH-independent solubility at pH of -2 to 14.0, In another embodiment, the neutral/non-ionizable compound has a pH-independent solubility at pH of -1 to 12.0. In another embodiment, the neutral/non-ionizable compound has a pH-independent solubility at pH of 0.0 to 10.0. In another embodiment, the neutral/or non-ionizable compound has a pH-independent solubility at pHs of 1.0 to 8.0. In another embodiment, the neutral or non-ionizable compound has a pH-independent solubility at pH of 1.0 to 8.0 and has a solubility of not more than 1 mg/mL at pH 1.0 to 8.0.

The API(s) that is (are) poorly water soluble are to be included in the pharmaceutical compositions of the present invention in a sufficient amount to be therapeutically effective in the system to be treated. The knowledge of therapeutically effective amounts for a given API is known to those skilled in the art.

For such compositions, care has to be taken to avoid that the API used completely or for a considerable portion precipitates, when exposed to the various aqueous environments of the GI tract. Rather, the formulation must act to temporarily increase the solubility of the drug, preferably at least one to multiple times the equilibrium solubility of said API in a relevant medium, allowing the API to be absorbed at the intended site in the GI tract.

As indicated above, the polymer solubilizer to be included in the present invention may comprise one or more components selected from the following classes: esters and lactones of organic acids, dicarboxylic acids and esters of dicarboxylic acids, or esters, ethers and carbonates of diols and triols and mixtures thereof. It will be appreciated that a polymer solubilizer may be referred to as a plasticizer.

In a preferred embodiment, the polymer solubilizer may include one or more esters and/or lactones of organic acids selected from diethyl malate, triethyl citrate, tributyl citrate, ethyl lactate, dimethyl succinate, diethyl succinate, glucuronolactone and D-(+)-glucuronic acid y-lactone.

In another or further preferred embodiment, the polymer solubilizer may include one or more dicarboxylic acids and/or esters of dicarboxylic acids selected from mono-methyl adipate, dimethyl glutarate and mono-methyl glutarate.

In other or further preferred embodiments, the polymer solubilizer may include one or more esters, ethers and carbonates of diols and/or triols selected from glycerol carbonate, propylene carbonate, ethylene carbonate, 1,2-butylene carbonate, glycerol formal, DL-1,2-isopropylideneglycerol, 1-butoxypropan-2-ol, tri(propylene glycol) methyl ether, dipropylene glycol methyl ether acetate, propylene glycol methyl ether acetate, dipropylene glycol methyl ether, 1-methoxy-2-propanol, diethylene glycol monoethyl ether, 3-methoxy-3-methyl-1-butanol, isosorbide dimethyl ether and dianhydro-d-glucitol.

As indicated above, the invention includes a glycol. Such a glycol can be selected from, but is not limited to, propylene glycol, dipropylene glycol, butylene glycol, glycerol, tetraglycol, 1,2-hexanediol, 1,2- butanediol, PEG 400 and polyglycerol. Preferably, the glycol comprises or consists of propylene glycol (PG).

The one or more glycols may be included at a molar ratio of between 12 to 1 and 1 to 10, preferably in the range between 12 to 1 and 1 to 4, more preferably in a range of between 12 to 1 to 1:2, even more preferably in the range of between 8 to 1 and 1 to 2, even more preferably in the range of between 8 to 1 and 1 to 1.5 and most preferably in a range of between 4 to 1 and 1 to 1; relative to the selected polymer solubilizer. When polymers of glycols are used, the molar ratio is determined based on the monomeric units, as described above.

The DES constituents, and preferably the NADES constituents, to be included in the liquid in the present invention will adhere to one of the following classes: organic acids, phenolic compounds, terpenoids, fatty acids, organic bases, sugars or sweeteners, glycols, amino acids, quaternary ammonium compounds, and derivatives of these classes.

In an embodiment, which may be preferred, the (NA)DES constituent may include one or more organic acids which may be one of, but not limited to, malic acid, citric acid, lactic acid, fumaric acid, tartaric acid, ascorbic acid, pimelic acid, gluconic acid, acetic acid and/or derivatives thereof such as nicotinamide. The one or more organic acids may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to the selected glycol or a polymer solubilizer component, as described above.

The (NA)DES constituent may further or alternatively include one or more phenolic compound which may be one of, but not limited to, propenyl guaethol isoeugenol, tocopherol, propyl gallate, tyramine, butyl paraben, vanillin. The one or more phenolic compound may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to the selected glycol or a polymer solubilizer component, as described above.

Additionally or alternatively, the (NA)DES constituent may include one or more terpenoid, which may be one of, but not limited to, terpineol, perillyl alcohol and menthol. The one or more terpenoid may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to a glycol, or a polymer solubilizer component as described above.

Yet further, or alternatively, the (NA)DES constituent may include one or more fatty acid, which may be one of, but not limited to, nonanic acid, octanoic acid, lauric acid, isopropyl myristate, ascorbyl palmitate. The one or more fatty acid may be included at a molar ratio between 0.1 to 1 and 4 to 1, preferably in the range between 0.1 to 1 and 2 to 1, more even preferably in the range between 0.2 to 1 and 1.75 to 1 and more preferably in the range between 0.3 to 1 and 1.5 to 1, relative to a glycol or a polymer solubilizer component, as described above.

In another embodiment, the (NA)DES constituent may include one or more organic base, which may be one of, but not limited to, urea and guanine. The one or more organic bases may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to a glycol or a polymer solubilizer component, as described above.

Further, the (NA)DES constituent may include one or more sugar or sweeteners selected from the group consisting of, but not limited to, sucrose, glucose, fructose, lactose, maltose, xylose, sucrose, inositol, xylitol, saccharin, sucralose, aspartame, acesulfame potassium and ribitol, as well as their phosphates. The one or more sugars or sweetener may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to the glycol or a polymer solubilizer component, as described above.

Additionally, the (NA)DES constituent may include one or more amino acids. Suitable amino acids may be selected from, but are not limited to, for example alanine, glutamic acid, glutamate, asparagine, aspartic acid, lysine, arginine, proline and threonine. The one or more amino acids may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to the glycol or a polymer solubilizer component, as described above.

In another embodiment, the (NA)DES constituent may include one or more quaternary ammonium compounds, which may be one of, but not limited to, choline chloride, thiamine mononitrate and carnitine. The one or more fatty acid may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to a glycol or a polymer solubilizer component, as described above.

In a further embodiment of the invention, the (NA)DES constituent may include more than one glycol. This may particularly be the case if the glycol comprises propylene glycol (PG). The additional glycol can be selected from, but is not limited to, dipropylene glycol, butylene glycol, glycerol and polyglycerol. The one or more additional glycols may be included at a molar ratio between 0.1 to 1 and 4 to 1, preferably in the range between 0.1 to 1 and 2 to 1, more preferably in the range between 0.2 to 1 and 1.75 to 1 and even more preferably in the range between 0.3 to 1 and 1.5 to 1, relative to PG. When polymers of glycols are used, the molar ratio is determined based on the monomeric units, as described above.

Modifications in the pH of the DES can be made with concentrated acids or bases, e.g. hydrochloric acid or sodium hydroxide.

The pharmaceutical composition is essentially intended for oral application. That is, it is intended that the API enters the system of a human or animal to be treated therewith via the gastrointestinal tract.

This makes that often, if not always, polymeric precipitation inhibitors (PPI) are to be incorporated in the DES of the invention.

Polymeric precipitation inhibitors (PPI) have been demonstrated to be useful and are broadly used in improving drug solubility and bioavailability of poorly water-soluble APIs in the gastrointestinal (GI) tract. In this respect reference is made to a publication by Vasconceles et al. in Drug Discovery Today, 2007, Vol 12, pages 1068-1075 and PPIs described there and/or in references therein, acting to reduce API precipitation and thereby creating a supersaturated state resulting in a boost in drug bioavailability of molecules capable of permeating the GI tract.

The invention is hence further directed to the solubilization of one or more pharmaceutically acceptable, biocompatible PPI's in the DES according to the invention. Preferably, one or more PPI('s) are solubilized at a mass ratio to the API of between 0.25 to 1 and 20 to 1. *E.g.,* if 10 mg of API is solubilized in 1 ml of the DES, between 2.5 and 200 mg of PPI are solubilized in the DES. In preferred embodiments of the DESs according to the invention, the weight ratio of PPI(s) to API(s) is between 0.2 to 1 and 20 to 1, between preferably 0.35 to 1 and 10 to 1, or more preferably between 0.5 to 1 and 5 to 1.

The PPI('s) are preferably solubilized in the DES after the combination of the selected glycol/polymer solubilizer fraction with the DES/NADES constituent(s); see herein-below.

The polymeric precipitation inhibitors useful in the present invention refer to polymers that are soluble in aqueous medium with pH range below 14. These may be ionic or neutral polymers with polar or charged functional groups. Preferably, the PPI is a water soluble polymer. Suitable PPI's may be selected from the group consisting of homopolymers and copolymers of N-vinyl lactams, especially homopolymers and copolymers of N-vinyl pyrrolidone, e.g. polyvinyl pyrrolidone (PVP), copolymers of N-vinyl pyrrolidone and vinyl acetate or vinyl propionate, polyvinyl caprolactam-polyvinyl acetate-polyethylene glycol graft copolymers, such as Soluplus^{®}, block copolymers of ethylene oxide and propylene oxide, also known as polyoxyethylene / polyoxypropylene block copolymers or polyoxyethylene polypropyleneglycol, such as Poloxamer^{®}, lauroyl polyoxyglycerides cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene oxide and polypropylene oxide and copolymers of ethylene oxide and propylene oxide, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, and mixtures of one or more thereof.

Essentially, preferred embodiments of the DESs of the present invention are composed of at least five components: glycol, a specific polymer solubilizer, a deep eutectic solvent (preferably a natural deep eutectic solvent) constituent, a polymeric precipitation inhibitor, and an active pharmaceutical ingredient.

In yet another preferred embodiment, the DESs of the invention also comprise a disintegrant. Disintegrants are polymers that increase the dissolution speed of a formulation within an aqueous environment. These components are commonly known and used in solid formulations to increase the dissolution speed but were found to perform the same function in viscous liquid formulations, like the DESs of the present invention. The disintegrant (if used) is homogenously mixed throughout the liquid DES formulation.

When used, disintegrants are included at a mass ratio of between 0.5% and 10%, drawn to the mass of the total DES composition.

Suitable disintegrants include, but are not limited to, Kollidon CL^{®} (BASF^{®}), microcrystalline cellulose, crospovidone, croscarmellose sodium, carboxymethyl cellulose, carboxymethyl cellulose calcium, carboxymethyl cellulose sodium, hydroxypropyl cellulose, methyl cellulose, chitosan, starch, sodium starch glycolate and mixtures thereof.

The constituents of the pharmaceutical formulations of the invention are preferably all GRAS (Generally Recognized As Safe) certified, but at least all are considered "pharmaceutically acceptable" at the relevant doses.

In a further aspect, the present invention relates to the use of a combination of a glycol with a polymer solubilizer component in the preparation of a deep eutectic solvent (DES) composition, preferably in the preparation of a pharmaceutical composition, the composition comprising a combination of a glycol with a polymer solubilizer component, which polymer solubilizer component is selected from a group consisting of esters and lactones of organic acids; dicarboxylic acids; esters of dicarboxylic acids; and esters, ethers and carbonates of diols and triols; and mixtures thereof, preferably a glycol and the polymer solubilizer component are used in a molar ratio of a glycol to the polymer solubilizer component in the range of between 12 to 1 and 1 to 10, preferably in the range between 12 to 1 and 1 to 2, preferably in a range of between 8 to 1 and 1 to 1.5, and more preferably in a range of between 4 to 1 and 1 to 1.

In another aspect, the invention relates to a process to prepare a pharmaceutical composition. This process comprises the following steps: a glycol and the specific polymer solubilizer are mixed to create a liquid, preferably a liquid core; one or more DES constituents, and preferably NADES constituents are added to said liquid forming a DES; and one or more APIs are solubilized in said DES. The DES constituents, as said herein-above, assist in increasing the solubility of the API of interest.

In a preferred embodiment of this process, one or more PPI's are subsequently solubilized in said pharmaceutical composition.

In yet a further preferred embodiment of the process of the invention, a disintegrant is mixed in said pharmaceutical composition.

The present invention will now be described in further detail with reference to the following working examples, which are intended not to limit the scope of the invention.

### Examples

In the examples below following abbreviations are used: TEC - Triethyl Citrate; PC - Propylene Carbonate; PG - Propylene Glycol; CA - Citric acid; MA - Malic acid; LA - Lactic acid; CC - Choline Chloride; Nic - Nicotinamide; TY - Tyramine; AC - Acetoin; TG - Tetraglycol; ASP - Ascorbyl Palmitate; DEM - Diethyl Malate; VA - Vanillin; BPA - Butyl Paraben; PEG - Polyethylene Glycol 400; PRG - Propyl Gallate; BG - Butylene Glycol; ME - Menthol; NL - Nerolidol; ISE - Isoeugenol; SAC - Saccharin; TRO - Tromentamine.

### Example 1

The solubilities of carvedilol, cyclosporine, flufenamic acid, ibuprofen, itraconazole and ketoconazole were determined in a range of DES formulations according to the invention. The DES systems contained propylene glycol, a specific polymer solubilizer, and one or more (NA)DES constituents. Table 1 provides an overview of tested drugs, their solubility in water, details on used DES compositions, and the solubility of the tested drug in the respective DES formulations.

**Table 1 - API solubility in tested liquids**

| **Active Pharmaceutical Ingredient** | | **DES** | | **Concentra tion solubilize d API (mg/mL)** |
|---|---|---|---|---|
| **Name** | **Water solubility (mg/mL) at 20 °C and pH 7.0** | **Constituents** | **Molar Ratio** | |
| Carvedilol | 0.6×10⁻³ | TEC:PG:LA | 0.5:2:1 | 200 |
| Carvedilol | 0.6×10⁻³ | TEC:PG:Nic | 0.5:2:0.5 | 75 |
| Carvedilol | 0.6×10⁻³ | TEC:PG:Urea | 0.5:2:0.5 | 50 |
| Carvedilol | 0.6×10⁻³ | TEC:PG:CC | 0.5:2:1 | 75 |
| Carvedilol | 0.6×10⁻³ | TEC:PG:MA | 0.5:2:1 | 200 |
| Carvedilol | 0.6×10⁻³ | TY:AC:TG:LA :DEM | 1:1:0.5:1:1 | >100 |
| Carvedilol | 0.6×10⁻³ | VA:AC:TG:LA :DEM | 1:1:0.5:1:1 | >100 |
| Carvedilol | 0.6×10⁻³ | VA:PRG:PEG: MA:EL | 1:1:0.5:1:1 | >100 |
| Carvedilol | 0.6×10⁻³ | ME:BPA:PEG :MA:EL | 1:1:0.5:1:1 | >100 |
| Carvedilol | 0.6×10⁻³ | PRG:BPA:BG: LA:DEM | 1:1:1:1:1 | >100 |
| Carvedilol | 0.6×10⁻³ | ISE:BPA:PG: DEM | 1:1:1:1 | >100 |
| Carvedilol | 0.6×10⁻³ | SAC:TRO:PG: PC | 1:1:1:1 | >100 |
| Cyclosporine | 0.3×10⁻³ | TEC:PG:LA | 0.5:2:1 | 200 |
| Cyclosporine | 0.3×10⁻³ | TEC:PG:Nic | 0.5:2:0.5 | 200 |
| Cyclosporine | 0.3×10⁻³ | TEC:PG:Urea | 0.5:2:0.5 | 200 |
| Cyclosporine | 0.3×10⁻³ | TEC:PG:CC | 0.5:2:1 | 75 |
| Cyclosporine | 0.3×10⁻³ | TEC:PG:MA | 0.5:2:1 | 25 |
| Cyclosporine | 0.3×10⁻³ | TY:AC:TG:LA :DEM | 1:1:0.5:1:1 | >100 |
| Cyclosporine | 0.3×10⁻³ | VA:AC:TG:LA :DEM | 1:1:0.5:1:1 | >100 |
| Cyclosporine | 0.3×10⁻³ | VA:PRG:PEG: MA:EL | 1:1:0.5:1:1 | >100 |
| Cyclosporine | 0.3×10⁻³ | ME:BPA:PEG :MA:EL | 1:1:0.5:1:1 | >100 |
| Cyclosporine | 0.3×10⁻³ | PRG:BPA:BG: LA:DEM | 1:1:1:1:1 | >100 |
| Cyclosporine | 0.3×10⁻³ | ME:NL:TG:L A:DEM | 1:1:1:1:1 | >50 |
| Cyclosporine | 0.3×10⁻³ | ISE:BPA:PG: DEM | 1:1:1:1 | >100 |
| Cyclosporine | 0,3×10⁻³ | SAC:TRO:PG: PC | 1:1:1:1 | >100 |
| Flufenamic Acid | 9.1×10⁻³ | TEC:PG:LA | 0.5:2:1 | 133 |
| Flufenamic Acid | 9.1×10⁻³ | TEC:PG:Nic | 0.5:2:0.5 | 200 |
| Flufenamic Acid | 9.1×10⁻³ | TEC:PG:Urea | 0.5:2:0.5 | 200 |
| Flufenamic Acid | 9.1×10⁻³ | TEC:PG:CC | 0.5:2:1 | 200 |
| Flufenamic Acid | 9.1×10⁻³ | TEC:PG:MA | 0.5:2:1 | 25 |
| Ibuprofen | 2.1×10⁻² | TEC:PG:LA | 0.5:2:1 | 200 |
| Ibuprofen | 2.1×10⁻² | TEC:PG:Nic | 0.5:2:0.5 | 200 |
| Ibuprofen | 2.1×10⁻² | TEC:PG:Urea | 0.5:2:0.5 | 200 |
| Ibuprofen | 2.1×10⁻² | TEC:PG:CC | 0.5:2:1 | 200 |
| Ibuprofen | 2.1×10⁻² | TEC:PG:MA | 0.5:2:1 | 25 |
| Carbamazepi ne | 1,7×10⁻² | TY:AC:TG:LA :DEM | 1:1:0.5:1:1 | >100 |
| Carbamazepi ne | 1,7×10⁻² | VA:AC:TG:LA :DEM | 1:1:0.5:1:1 | >100 |
| Carbamazepi ne | 1,7×10⁻² | VA:PRG:PEG: MA:EL | 1:1:0.5:1:1 | >100 |
| Carbamazepi ne | 1,7×10⁻² | PRG:BPA:BG: LA:DEM | 1:1:1:1:1 | >100 |
| Carbamazepi ne | 1,7×10⁻² | ISE:BPA:PG: DEM | 1:1:1:1 | >50 |
| Carbamazepi ne | 1,7×10⁻² | SAC:TRO:PG: PC | 1:1:1:1 | >50 |
| Itraconazole | 4.0×10⁻⁶ | TEC:PG:CA: MA | 0.5:2:0.75:0 .75 | 75 |
| Itraconazole | 4.0×10⁻⁶ | TEC:PG:MA | 0.5:2:1 | 75 |
| Itraconazole | 4.0×10⁻⁶ | PC:PG:MA | 0.5:2:1 | 50 |
| Itraconazole | 4.0×10⁻⁶ | PC:PG:CA:M A | 0.5:2:0.75:0 .75 | 150 |
| Itraconazole | 4.0×10⁻⁶ | PC:PG:CA | 0.5:2:1 | 150 |
| Itraconazole | 4.0×10⁻⁶ | VA:AC:TG:LA :DEM | 1:1:0.5:1:1 | >100 |
| Itraconazole | 4.0×10⁻⁶ | VA:PRG:PEG: MA:EL | 1:1:0.5:1:1 | >100 |
| Itraconazole | 4.0×10⁻⁶ | PRG:BPA:BG: LA:DEM | 1:1:1:1:1 | >100 |
| Itraconazole | 4.0×10⁻⁶ | ISE:BPA:PG: DEM | 1:1:1:1 | >50 |
| Ketoconazole | 9.0×10⁻⁵ | TEC:PG:LA | 0.5:2:1 | 200 |
| Ketoconazole | 9.0×10⁻⁵ | TEC:PG:Nic | 0.5:2:0.5 | 75 |
| Ketoconazole | 9.0×10⁻⁵ | TEC:PG:Urea | 0.5:2:0.5 | 25 |
| Ketoconazole | 9.0×10⁻⁵ | TEC:PG:CC | 0.5:2:1 | 25 |
| Ketoconazole | 9.0×10⁻⁵ | TEC:PG:MA | 0.5:2:1 | 200 |
| Ketoconazole | 9.0×10⁻⁵ | TY:AC:TG:LA :DEM | 1:1:0.5:1:1 | >100 |
| Ketoconazole | 9.0×10⁻⁵ | TY:ASP:TG:L A:DEM | 1:1:0.5:1:1 | >100 |
| Ketoconazole | 9.0×10⁻⁵ | VA:AC:TG:LA :DEM | 1:1:0.5:1:1 | >100 |
| Ketoconazole | 9.0×10⁻⁵ | VA:BP:PEG: MA:EL | 1:1:0.5:1:1 | >100 |
| Ketoconazole | 9.0×10⁻⁵ | VA:PRG:PEG: MA:EL | 1:1:0.5:1:1 | >100 |
| Ketoconazole | 9.0×10⁻⁵ | ME:BPA:PEG :MA:EL | 1:1:0.5:1:1 | >100 |
| Ketoconazole | 9.0×10⁻⁵ | PRG:BPA:BG: LA:DEM | 1:1:1:1:1 | >100 |
| Ketoconazole | 9.0×10⁻⁵ | ISE:BPA:PG: DEM | 1:1:1:1 | >50 |
| Ketoconazole | 9.0×10⁻⁵ | SAC:TRO:PG: PC | 1:1:1:1 | >100 |

From the data in Table 1 it is clear that all tested DES formulations are suitable to solubilize tested drugs in a concentration exceeding the solubility of that drug in water by a factor of at least 100 for ibuprofen, up to a factor of 10.000.000 for itraconazole.

As can be seen in Table 1, itraconazole was found to be soluble in a concentration of up to 200 mg/mL in the exemplary DES comprising propylene glycol, propylene carbonate, citric acid and malic acid at a molar ratio of 2:0.5:0.75:0.75. In a propylene glycol, propylene carbonate DES, with no NADES constituents, at a molar ration of 2:0.5, the solubility of itraconazole was poor (<25mg/ml). This finding indicates the effect of the addition of NADES constituents.

### Example 2

Table 2 shows the determined minimum solubility of a broad range of polymeric precipitation inhibitors in exemplary DES systems according to Table 1.

As shown, the DES liquids are capable of solubilizing large concentrations of a broad range of PPI's.

The breadth in both API solubility as demonstrated in Example 1 and PPI solubility contribute to the performance of API formulations according to the invention.

**Table 2: solubility (mg/mL) of polymeric precipitation inhibitors (PPI) in a number of DES formulations**

| **Polymeric Precipitation Inhibitor** | **DES Constituen ts** | **DES Composition Molar Ratio** | **Solubilize d PPI (mg/mL)** |
|---|---|---|---|
| Sigma Aldrich^{®} now Merck ^{®} - Cellulose Acetate Phthalate. Prod. No. 22192. | TEC:PG: MA | 0.5:2:1 | >85 |
| Ashland^{®} - AquaSolve^{®} HPMC-AS L Grade | TEC:PG: MA | 0.5:2:1 | >50 |
| Ashland^{®} - AquaSolve^{®} HPMC-AS M Grade | TEC:PG: MA | 0.5:2:1 | >50 |
| Ashland^{®} - AquaSolve^{®} HPMC-AS H Grade | TEC:PG: MA | 0.5:2:1 | >50 |
| Ashland^{®} - Klucell^{®} - HXF PHARM | TEC:PG: MA | 0.5:2:1 | >50 |
| Dow Chemical^{®} - AFFINISOL^{™} HPMC HME 4M | TEC:PG: MA | 0.5:2:1 | >50 |
| Dow Chemical^{®} - AFFINISOL^{®} HPMC HME 15LV | TEC:PG: MA | 0.5:2:1 | >50 |
| Alfa Aesar^{®} Hydroxypropyl methylcellulose Powder | TEC:PG: MA | 0.5:2:1 | >50 |
| BASF^{®} - Soluplus Polyvinyl caprolactame - polyvinyl acetate- polyethylene glycol graft copolymer | TEC:PG: MA | 0.5:2:1 | >50 |
| Sigma Aldrich^{®} now Merck^{®} - Polyvinylpyrrolidone K90, powder, , average M_{w} 360.000 g/mol. Prod. No. 81440. | TEC:PG: MA | 0.5:2:1 | >50 |
| Sigma Aldrich^{®} now Merck ^{®} - Poly(ethylene Glycol) M_{w} 8.000 g/mol. Prod No. P89510 | TEC:PG: MA | 0.5:2:1 | >50 |
| Sigma Aldrich^{®} now Merck ^{®} - Poly(ethylene Glycol) M_{w} 200 g/mol. Prod. No. P88440 | TEC:PG: MA | 0.5:2:1 | >50 |
| Sigma Aldrich^{®} now Merck ^{®} - Methyl-2- Hydroxyethyl Cellulose. Prod. No. 435015 | TEC:PG: MA | 0.5:2:1 | >50 |
| Sigma Aldrich^{®} now Merck ^{®} - METHOCEL^{®} A15 LV. Prod. No. P64605 | TEC:PG: MA | 0.5:2:1 | >50 |
| Ashland^{®} - BENECEL^{®} E4M PHARM | TEC:PG: MA | 0.5:2:1 | >50 |
| Evonik^{®} - EUDRAGIT^{®} E100 | TEC:PG: MA | 0.5:2:1 | >50 |
| BASF^{®} - Kollidon^{®} VA 64 | TEC:PG: MA | 0.5:2:1 | >50 |
| Sigma Aldrich^{®} now Merck^{®} - Tween^{®} 80. Prod. No. P4780 | TEC:PG: MA | 0.5:2:1 | >50 |
| Sigma Aldrich^{®} now Merck ^{®} - Cellulose Acetate Phthalate. Prod. No. 22192. | PC:PG:CA:M A | 0.5:2:0.75:0.75 | >50 |
| Ashland^{®} - AquaSolve^{®} HPMC-AS L Grade | PC:PG:CA:M A | 0.5:2:0.75:0.75 | >50 |
| Ashland^{®} - AquaSolve^{®} HPMC-AS M Grade | PC:PG:CA:M A | 0.5:2:0.75:0.75 | >50 |
| Ashland^{®} - AquaSolve^{®} HPMC-AS H Grade | PC:PG:CA:M A | 0.5:2:0.75:0.75 | >50 |
| Ashland^{®} - Klucell^{®} - HXF PHARM | PC:PG:CA:M A | 0.5:2:0.75:0.75 | >50 |
| Dow Chemical - AFFINISOL^{™} HPMC HME 4M | PC:PG:CA:M A | 0.5:2:0.75:0.75 | >50 |
| Dow Chemical^{®} - AFFINISOL^{®} HPMC HME 15LV | PC:PG:CA:M A | 0.5:2:0.75:0.75 | >50 |
| Alfa Aesar^{®} Hydroxypropyl methylcellulose Powder CAS: 9004-65-3 | PC:PG:CA:M A | 0.5:2:0.75:0.75 | >50 |
| BASF^{®} - Soluplus Polyvinyl caprolactame - polyvinyl acetate- polyethylene glycol graft copolymer | PC:PG:CA:M A | 0.5:2:0.75:0.75 | >50 |
| Sigma Aldrich^{®} now Merck^{®} - Polyvinylpyrrolidone K90, powder, average M_{w} 360.000 g/mol. Prod. No. 81440. | PC:PG:CA:M A | 0.5:2:0.75:0.75 | >50 |
| Sigma Aldrich^{®} now Merck ^{®} - Poly(ethylene Glycol) M_{w} 8.000 g/mol. Prod No. P89510 | PC:PG:CA:M A | 0.5:2:0.75:0.75 | >50 |
| Sigma Aldrich^{®} now Merck ^{®} - Poly(ethylene Glycol) M_{w} 200 g/mol. Prod. No. P88440 | PC:PG:CA:M A | 0.5:2:0.75:0.75 | >50 |
| Sigma Aldrich^{®} now Merck ^{®} - Methyl-2- Hydroxyethyl Cellulose. Prod. No. 435015 | PC:PG:CA:M A | 0.5:2:0.75:0.75 | >50 |
| Sigma Aldrich^{®} now Merck ^{®} - METHOCEL^{®} A15 LV. Prod. No. 64605 | PC:PG:CA:M A | 0.5:2:0.75:0.75 | >50 |
| Ashland^{®} - BENECEL^{®} E4M PHARM | PC:PG:CA:M A | 0.5:2:0.75:0.75 | >50 |
| Evonik^{®} - EUDRAGIT^{®} E100 | PC:PG:CA:M A | 0.5:2:0.75:0.75 | >50 |
| BASF^{®} - Kollidon^{®} VA 64 | PC:PG:CA:M A | 0.5:2:0.75:0.75 | >50 |
| Sigma Aldrich^{®} now Merck^{®} - Tween^{®} 80. Prod. No. P4780 | PC:PG:CA:M A | 0.5:2:0.75:0.75 | >50 |
| Dow Chemical^{®} - AFFINISOL^{®} HPMC HME 15LV | VA:AC:TG:L A:DEM | 1:1:0.5:1:1 | >50 |
| Dow Chemical^{®} - AFFINISOL^{®} HPMC HME 15LV | VA:BP:PEG: MA:EL | 1:1:0.5:1:1 | >50 |
| Dow Chemical^{®} - AFFINISOL^{®} HPMC HME 15LV | VA:PRG:PE G:MA:EL | 1:1:0.5:1:1 | >50 |
| Dow Chemical^{®} - AFFINISOL^{®} HPMC HME 15LV | ME:BP A:PE G:MA:EL | 1:1:0.5:1:1 | >50 |
| Dow Chemical^{®} - AFFINISOL^{®} HPMC HME 15LV | ME:NL:TG:L A:DEM | 1:1:1:1:1 | >50 |
| Dow Chemical^{®} - AFFINISOL^{®} HPMC HME 15LV | PRG:BPA:B G:LA:DEM | 1:1:1:1:1 | >50 |
| Dow Chemical^{®} - AFFINISOL^{®} HPMC HME 15LV | ISE:BPA:PG :DEM | 1:1:1:1 | >50 |
| BASF^{®} - Kollidon^{®} VA 64 | TY:ASP:TG: LA:DEM | 1:1:0.5:1:1 | >50 |
| BASF^{®} - Kollidon^{®} VA 64 | VA:AC:TG:L A:DEM | 1:1:0.5:1:1 | >50 |
| BASF^{®} - Kollidon^{®} VA 64 | VA:BP:PEG: MA:EL | 1:1:0.5:1:1 | >50 |
| BASF^{®} - Kollidon^{®} VA 64 | VA:PRG:PE G:MA:EL | 1:1:0.5:1:1 | >50 |
| BASF^{®} - Kollidon^{®} VA 64 | ME:BP A:PE G:MA:EL | 1:1:0.5:1:1 | >50 |
| BASF^{®} - Kollidon^{®} VA 64 | ME:NL:TG:L A:DEM | 1:1:1:1:1 | >50 |
| BASF^{®} - Kollidon^{®} VA 64 | PRG:BPA:B G:LA:DEM | 1:1:1:1:1 | >50 |
| BASF^{®} - Kollidon^{®} VA 64 | ISE:BPA:PG :DEM | 1:1:1:1 | >50 |
| BASF^{®} - Kollidon^{®} VA 64 | SAC:TRO:P G:PC | 1:1:1:1 | >50 |
| BASF^{®} - Soluplus Polyvinyl caprolactame - polyvinyl acetate- polyethylene glycol graft copolymer | TY:AC:TG:L A:DEM | 1:1:0.5:1:1 | >50 |
| BASF^{®} - Soluplus Polyvinyl caprolactame - polyvinyl acetate- polyethylene glycol graft copolymer | TY:ASP:TG: LA:DEM | 1:1:0.5:1:1 | >50 |
| BASF^{®} - Soluplus Polyvinyl caprolactame - polyvinyl acetate- polyethylene glycol graft copolymer | VA:AC:TG:L A:DEM | 1:1:0.5:1:1 | >50 |
| BASF^{®} - Soluplus Polyvinyl caprolactame - polyvinyl acetate- polyethylene glycol graft copolymer | VA:BP:PEG: MA:EL | 1:1:0.5:1:1 | >50 |
| BASF^{®} - Soluplus Polyvinyl caprolactame - polyvinyl acetate- polyethylene glycol graft copolymer | VA:PRG:PE G:MA:EL | 1:1:0.5:1:1 | >50 |
| BASF^{®} - Soluplus Polyvinyl caprolactame - polyvinyl acetate- polyethylene glycol graft copolymer | ME:BP A:PE G:MA:EL | 1:1:0.5:1:1 | >50 |
| BASF^{®} - Soluplus Polyvinyl caprolactame - polyvinyl | ME:NL:TG:L A:DEM | 1:1:1:1:1 | >50 |
| acetate- polyethylene glycol graft copolymer | | | |
| BASF^{®} - Soluplus Polyvinyl caprolactame - polyvinyl acetate- polyethylene glycol graft copolymer | PRG:BPA:B G:LA:DEM | 1:1:1:1:1 | >50 |
| BASF^{®} - Soluplus Polyvinyl caprolactame - polyvinyl acetate- polyethylene glycol graft copolymer | ISE:BPA:PG :DEM | 1:1:1:1 | >50 |
| BASF^{®} - Soluplus Polyvinyl caprolactame - polyvinyl acetate- polyethylene glycol graft copolymer | SAC:TRO:P G:PC | 1:1:1:1 | >50 |
| Evonik^{®} - EUDRAGIT^{®} E100 | VA:AC:TG:L A:DEM | 1:1:0.5:1:1 | >50 |
| Evonik^{®} - EUDRAGIT^{®} E100 | VA:BP:PEG: MA:EL | 1:1:0.5:1:1 | >50 |
| Evonik^{®} - EUDRAGIT^{®} E100 | VA:PRG:PE G:MA:EL | 1:1:0.5:1:1 | >50 |
| Evonik^{®} - EUDRAGIT^{®} E100 | ME:BPA:PE G:MA:EL | 1:1:0.5:1:1 | >50 |
| Evonik^{®} - EUDRAGIT^{®} E100 | ME:NL:TG:L A:DEM | 1:1:1:1:1 | >50 |
| Evonik^{®} - EUDRAGIT^{®} E100 | PRG:BPA:B G:LA:DEM | 1:1:1:1:1 | >50 |
| Evonik^{®} - EUDRAGIT^{®} E100 | ISE:BPA:PG :DEM | 1:1:1:1 | >50 |
| Ashland^{®} - Klucell^{®} - HXF PHARM | VA:BP:PEG: MA:EL | 1:1:0.5:1:1 | >50 |
| Ashland^{®} - Klucell^{®} - HXF PHARM | VA:PRG:PE G:MA:EL | 1:1:0.5:1:1 | >50 |

### Example 3

To a DES formulation comprising propylene glycol, propylene carbonate, citric acid and malic acid at a molar ratio of 2:0.5:0.75:0.75 itraconazole was added at a concentration of 50 mg/ml. To the DES formulation comprising itraconazole in a concentration of 50 mg/mL a number of polymeric precipitation inhibitors (PPIs) were added to form a formulation according to the invention. To sample 1 polyvinyl pyrrolidone/vinyl acetate (BASF^{®} - Kollidon^{®} VA64) and hydroxypropyl methylcellulose hot melt extrusion 15 LV (Dow Chemical^{®} - AFFINISOL^{®} HPMC HME 15LV) were at a mass ratio PPI to itraconazole of 0.9 to 0.9 to 1. To sample 2 the same PPIs were added at a mass ratio PPI to itraconazole of in 1.2 to 1.2 to 1.

API dissolution properties of sample 1 (S1) and sample 2 (S2) along with a comparative sample (S3) without PPIs were determined according to the standardized USP Dissolution Method, using apparatus 2 (^{©} 2001 The United States Pharmacopeia Convention).

Table 3 provides an overview of the composition of samples 1 (S1) and 2 (S2) and the comparative formulation (S3) without PPIs.

**Table 3: Composition of exemplary liquid formulations comprising itraconazole.**

| | | **PPI** | |
|---|---|---|---|
| | itraconazole | BASF^{®} - Kollidon^{®} VA64 | Dow Chemical^{®} - AFFINISOL^{®} HPMC HME 15LV |
| S1 | 50 mg/mL | 45 mg/mL | 45 mg/mL |
| S2 | 50 mg/mL | 60 mg/mL | 60 mg/mL |
| S3 | 50 mg/mL | - | - |

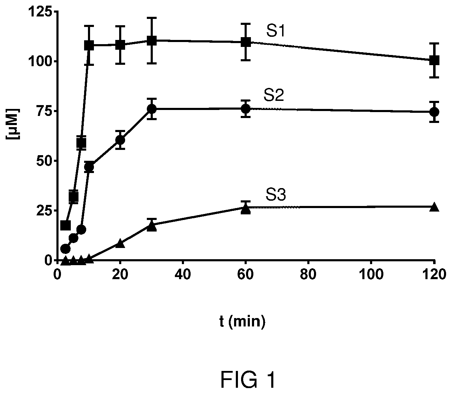

The performance of the polymeric precipitation inhibitors (PPI) was analyzed in accordance with USP dissolution method 2. According to this method the samples with the highest concentration of itraconazole in the dissolution vessel over time were classified as best performers. FIG 1 depicts the recorded concentration of itraconazole in micromoles/liter in the dissolution vessel as a function of time in minutes for samples S1, S2, and comparative sample S3. The two formulations according to the invention achieve a higher concentration of itraconazole and maintain this higher concentration over time. This indicates improved drug solubility over time with formulations produced with the platform of the invention.

### Example 4

Two itraconazole formulations according to the invention were selected to evaluate the *in vivo* bio availability of the API in comparison to a commercial itraconazole formulation, Sporanox^{®} (ex Janssen-Cilag SpA; Italy).

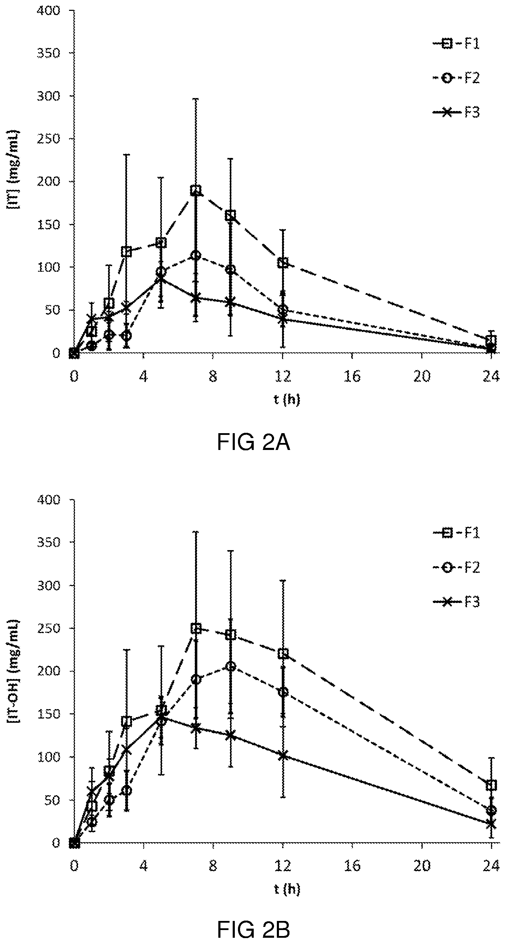

For this purpose, fasted male Sprague-Dawley rats (n = 3) with a mass of ~300 g, were dosed through oral gavage with 1.5 mg of formulation 1 (F1), formulation 2 (F2), and comparative formulation 3 (F3) respectively. After dosing, the plasma levels of the API and its metabolite hydroxyl-itraconazole were determined as a function of time. Samples were withdrawn 0, 1, 2, 3, 5, 7, 9, 12 and 24 hours after administering the formulations. API levels were determined by Liquid Chromatography-Mass Spectrometry (LC-MS). FIG. 2A depicts the mean measured plasma levels (mg/mL) of itraconazole (IT). FIG. 2B depicts the mean measured plasma levels (mg/mL) of hydroxyl- itraconazole (IT-OH).

From FIG. 2 it can be observed that formulations 1 and 2 show improved bioavailability of itraconazole over the comparative formulation as indicated by the mean plasma levels of itraconazole and hydroxyl- itraconazole in rats dosed with formulation 1, respectively formulation 2, exceeds the plasma levels of the respective APIs in rats dosed with the comparative formulation.

### Comparative Example 1

Composition as shown in Table 4 were prepared. These compositions each comprise citric acid and other constituents and are based on Examples 1,2,3,5, 8 and 9 as disclosed in WO 97/00670 and were found to be highly viscous at room temperature. To each composition, the polymeric precipitation inhibitor HPMC polymer (Dow Chemical^{®} - AFFINISOL^{®} HPMC HME 15LV) was added in an amount of 50 mg/ml to test its solubility in each composition. It was found that the HPMC polymer was insoluble at this concentration in each composition.

**Table 4: solubility tests of HPMC in comparative compositions**

| **Comparative composition** | **Corresponding example in** WO 97/00670 | **Constituents (g)** | **Solubility of 50 mg/ml HPMC polymer** |
|---|---|---|---|
| 1 | 1 | citric acid (4 g) | insoluble |
| | | glycerol (4 g) | |
| 2 | 2 | citric acid ( 4g) | insoluble |
| | | propylene glycol (5.5 g) | |
| 3 | 3 | citric acid (5.1 g) | insoluble |
| | | propylene glycol (4.1 g) | |
| | | oleyl alcohol (0.19 g) | |
| | | Brij 98 (0.14 g) | |
| 4 | 5 | citric acid (5.9 g) | insoluble |
| | | propylene glycol (3.7 g) | |
| | | oleoyl alcohol (0.2 g) | |
| | | Brij 98 (0.2 g) | |
| 5 | 8 | citric acid (4 g) | insoluble |
| | | propylene glycol (2.6 g) | |
| | | glycerol (2.6 g) | |
| | | foaming agent (0.25 g) | |
| | | foam stabilizer (0.75 g) | |
| 6 | 9 | citric acid (4 g) | insoluble |
| | | glycerol (4 g) | |
| | | trimethanolamine (2 g) | |

### Example 5

To each comparative composition described in Comparative Example 1, a polymer solubilizing component was added in an amount as indicated in Table 5. Then, to each modified composition, the polymeric precipitation inhibitor HPMC polymer (Dow Chemical^{®} - AFFINISOL^{®} HPMC HME 15LV) was added in an amount of 50 mg/ml to test its solubility in each modified composition. It was found that the HPMC polymer was soluble at this concentration in each modified composition.

**Table 5: solubility tests of HPMC in modified comparative compositions**

| **Comparative composition** | **Additional polymer solubilizing component added (wt%)** | **Solubility of 50 mg/ml HPMC polymer** |
|---|---|---|
| 1 | diethyl malate (40%) | soluble |
| 2 | diethyl malate (25%) | soluble |
| 3 | diethyl malate (25%) | soluble |
| 4 | diethyl malate (25%) | soluble |
| 5 | diethyl malate (25%) | soluble |
| 6 | diethyl malate (40%) | soluble |

## Claims

1. A deep eutectic solvent (DES) composition, comprising a combination of a glycol with a polymer solubilizer component, which polymer solubilizer component is selected from the group consisting of esters and lactones of organic acids; dicarboxylic acids; esters of dicarboxylic acids; esters, ethers and carbonates of diols and triols; and mixtures thereof, in a molar ratio of a glycol to the polymer solubilizer component in the range of between 12 to 1 and 1 to 10, preferably in a range of between 8 to 1 and 1 to 2, and more preferably in a range of between 4 to 1 and 1 to 1; the composition further comprising at least one DES constituent.

2. The DES composition according to claim 1 wherein the glycol is selected from the group consisting of propylene glycol, dipropylene glycol, butylene glycol, glycerol, tetraglycol, 1,2-hexanediol, 1,2-butanediol, PEG 400 and polyglycerol, preferably propylene glycol.

3. The DES composition according to claim 1, wherein the DES constituent comprises at least one naturally ocurring deep eutectic solvent (NADES) constituent.

4. The DES composition according to any of the preceding claims, wherein the DES constituent is selected from the group consisting of organic acids, phenolic compounds, terpenoids, fatty acids, organic bases, sugars or sweeteners, glycols, amino acids, quaternary ammonium compounds such as choline compounds, and derivatives of these classes.

5. The DES composition according to claim 1, including one or more of: an organic acid which may be one of malic acid, citric acid, lactic acid, fumaric acid, tartaric acid, ascorbic acid, pimelic acid, gluconic acid and acetic acid and/or derivatives thereof such as nicotinamide; one or more phenolic compound, which may be one of propenyl guaethol isoeugenol, tocopherol, propyl gallate, tyramine, butyl paraben and vanillin; one or more terpenoid, which may be one of terpineol, perillyl alcohol and menthol; one or more fatty acid, which may be one of nonanic acid, octanoic acid, lauric acid, isopropyl myristate, ascorbyl palmitate; one or more organic base, which may be one of urea and guanine; a sugar or sweetener which may be one of sucrose, glucose, fructose or lactose; an amino acid which may be one of alanine, glutamic acid or glutamate; a quaternary ammonium compounds which may be choline chloride, thiamine mononitrate or carnitine.

6. The DES composition according to any of the preceding claims, wherein the DES/NADES constituent(s) are provided at a molar ratio in the range between 0.1 to 1 and 4 to 1 for each constituent relative to the polymer solubilizer component, preferably in the range between 0.2 to 1 and 1.75 to 1 and more preferably in the range between 0.3 to 1 and 1.5 to 1.

7. The DES composition according to any one of the preceding claims, further comprising an active pharmaceutical ingredient (API).

8. The DES composition according to any one of the preceding claims, further comprising one or more polymeric precipitation inhibitors (PPIs).

9. The DES composition according to claim 8, wherein the weight ratio of PPI(s) to API(s) is between 0.2 to 1 and 20 to 1, between preferably 0.35 to 1 and 10 to 1, or more preferably between 0.5 to 1 and 5 to 1.

10. The DES composition according to any one of the preceding claims further comprising one or more disintegrants, preferably are present at a mass percentage between 0.25 and 10%, drawn to the mass of the total DES.

11. The DES composition according to any one of the preceding claims for oral administration,

12. A process to prepare a pharmaceutical composition, comprising the following steps: mixing a glycol and the polymer solubilizer as defined in claim 1 to create a liquid; adding one or more DES constituents, and preferably NADES constituents, to said liquid forming a DES; and solubilizing one or more APIs in said DES.

13. The process of claim 12, wherein one or more PPIs are solubilized in said pharmaceutical composition.

14. The process of claim 12 or claim 13, wherein a disintegrant is mixed in said pharmaceutical composition.

15. Use of a combination of a glycol with a polymer solubilizer component in the preparation of a deep eutectic solvent (DES) composition, wherein the polymer solubilizer component is selected from the group consisting of esters and lactones of organic acids; dicarboxylic acids; esters of dicarboxylic acids; esters, ethers and carbonates of diols and triols; and mixtures thereof, preferably wherein the glycol and the polymer solubilizer component are used in a molar ratio in the range of between 12 to 1 and 1 to 10, more preferably in a range of between 8 to 1 and 1 to 1.5, and most preferably in a range of between 4 to 1 and 1 to 1.

## Patentansprüche

1. Zusammensetzung von tiefeneutektischem Lösungsmittel (DES), die eine Kombination eines Glykols mit einer Polymerlösungsvermittlerkomponente umfasst, wobei die Polymerlösungsvermittlerkomponente aus der Gruppe ausgewählt ist, bestehend aus Estern und Lactonen von organischen Säuren; Dicarbonsäuren; Estern von Dicarbonsäuren; Estern, Ethern und Carbonaten von Diolen und Triolen; und Mischungen davon, in einem molaren Verhältnis eines Glykols zu der Polymerlösungsvermittlerkomponente im Bereich zwischen 12 zu 1 und 1 zu 10, vorzugsweise in einem Bereich zwischen 8 zu 1 und 1 zu 2, und bevorzugter in einem Bereich zwischen 4 zu 1 und 1 zu 1; wobei die Zusammensetzung ferner wenigstens einen DES-Bestandteil umfasst.

2. DES-Zusammensetzung nach Anspruch 1, wobei das Glykol aus der Gruppe ausgewählt ist, bestehend aus Propylenglykol, Dipropylenglykol, Butylenglykol, Glycerin, Tetraglykol, 1,2-Hexandiol, 1,2-Butandiol, PEG 400 und Polyglycerin, vorzugsweise Propylenglykol.

3. DES-Zusammensetzung nach Anspruch 1, wobei der DES-Bestandteil wenigstens einen natürlich vorkommenden Bestandteil von tiefeneutektischem Lösungsmittel (NADES) umfasst.

4. DES-Zusammensetzung nach einem der vorhergehenden Ansprüche, wobei der DES-Bestandteil aus der Gruppe ausgewählt ist, bestehend aus organischen Säuren, phenolischen Verbindungen, Terpenoiden, Fettsäuren, organischen Basen, Zuckern oder Süßungsmitteln, Glykolen, Aminosäuren, quaternären Ammoniumverbindungen wie Cholinverbindungen und Derivaten dieser Klassen.

5. DES-Zusammensetzung nach Anspruch 1, die einschließt: eine organische Säure, die eine Äpfelsäure und/oder Zitronensäure und/oder Milchsäure und/oder Fumarsäure und/oder Weinsäure und/oder Ascorbinsäure und/oder Pimelinsäure und/oder Gluconsäure und/oder Essigsäure und/oder Derivate davon, wie Nicotinamid sein kann; und/oder eine oder mehrere phenolische Verbindungen, die Propenylguaethol-Isoeugenol und/oder Tocopherol und/oder Propylgallat und/oder Tyramin und/oder Butylparaben und/oder Vanillin sein können; und/oder ein oder mehrere Terpenoide, die Terpineol und/oder Perillylalkohol und/oder Menthol sein können; und/oder eine oder mehrere Fettsäuren, die Nonansäure und/oder Octansäure, Laurinsäure und/oder Isopropylmyristat und/oder Ascorbylpalmitat sein kann; und/oder eine oder mehrere organische Basen, die Harnstoff und/oder Guanin sein kann; einen Zucker oder Süßstoff, der Saccharose und/oder Glucose und/oder Fructose und/oder Lactose sein kann; und/oder eine Aminosäure, die Alanin und/oder Glutaminsäure und/oder Glutamat sein kann; eine quaternäre Ammoniumverbindung, die Cholinchlorid und/oder Thiaminmononitrat und/oder Carnitin sein kann.

6. DES-Zusammensetzung nach einem der vorhergehenden Ansprüche, wobei der/die DES/NADES-Bestandteil(e) in einem molaren Verhältnis im Bereich zwischen 0,1 zu 1 und 4 zu 1 für jeden Bestandteil relativ zur Polymerlösungsvermittlerkomponente bereitgestellt wird/werden, vorzugsweise im Bereich zwischen 0,2 zu 1 und 1,75 zu 1, und bevorzugter im Bereich zwischen 0,3 zu 1 und 1,5 zu 1.

7. DES-Zusammensetzung nach einem der vorhergehenden Ansprüche, die ferner einen pharmazeutischen Wirkstoff (API) umfasst.

8. DES-Zusammensetzung nach einem der vorhergehenden Ansprüche, die ferner einen oder mehrere polymere Ausfällungsinhibitoren (PPI) umfasst.

9. DES-Zusammensetzung nach Anspruch 8, wobei das Gewichtsverhältnis von PPI(s) zu API(s) zwischen 0,2 zu 1 und 20 zu 1, vorzugsweise zwischen 0,35 zu 1 und 10 zu 1, oder bevorzugter zwischen 0,5 zu 1 und 5 zu 1 liegt.

10. DES-Zusammensetzung nach einem der vorhergehenden Ansprüche, die ferner ein oder mehrere Auflösungsmittel umfasst, die vorzugsweise in einem Massenanteil zwischen 0,25 und 10 %, bezogen auf die Masse des gesamten DES, vorhanden sind.

11. DES-Zusammensetzung nach einem der vorhergehenden Ansprüche zur oralen Verabreichung.

12. Verfahren zur Herstellung einer pharmazeutischen Zusammensetzung, das die folgenden Schritte umfasst: Mischen eines Glykols und des in Anspruch 1 definierten Polymerlösungsvermittlers, um eine Flüssigkeit zu erzeugen; Hinzufügen eines oder mehrerer DES-Bestandteile und vorzugsweise NADES-Bestandteile zu der Flüssigkeit, wobei ein DES gebildet wird; und Solubilisieren eines oder mehrerer APIs in dem DES.

13. Verfahren nach Anspruch 12, wobei ein oder mehrere PPIs in der pharmazeutischen Zusammensetzung solubilisiert sind.

14. Verfahren nach Anspruch 12 oder 13, wobei der pharmazeutischen Zusammensetzung ein Auflösungsmittel beigemischt wird.

15. Verwendung einer Kombination eines Glykols mit einer Polymerlösungsvermittlerkomponente bei der Herstellung einer Zusammensetzung von tiefeneutektischem Lösungsmittel (DES), wobei die Polymerlösungsvermittlerkomponente aus der Gruppe ausgewählt ist, bestehend aus Estern und Lactonen von organischen Säuren; Dicarbonsäuren; Dicarbonsäuren; Estern von Dicarbonsäuren; Estern, Ethern und Carbonaten von Diolen und Triolen; und Mischungen davon, wobei das Glykol und die Polymerlösungsvermittlerkomponente vorzugsweise in einem molaren Verhältnis im Bereich zwischen 12 zu 1 und 1 zu 10, bevorzugter in einem Bereich zwischen 8 zu 1 und 1 zu 1,5 verwendet werden, und am meisten bevorzugt in einem Bereich zwischen 4 bis 1 und 1 bis 1 verwendet werden.

## Revendications

1. Composition de solvant eutectique profond (DES), comprenant une combinaison d'un glycol avec un composant solubilisant polymère, lequel composant solubilisant polymère est choisi dans le groupe constitué d'esters et de lactones d'acides organiques ; d'acides dicarboxyliques ; d'esters d'acides dicarboxyliques ; d'esters, d'éthers et de carbonates de diols et de triols ; et de mélanges de ceux-ci, selon un rapport molaire d'un glycol au composant solubilisant polymère dans la plage entre 12 à 1 et de 1 à 10, de préférence dans la plage entre 8 à 1 et 1 à 2, et de manière plus préférée dans la plage entre 4 à 1 et 1 à 1 ; la composition comprenant en outre au moins un constituant DES.

2. Composition de DES selon la revendication 1, dans laquelle le glycol est choisi dans le groupe constitué de propylène glycol, de dipropylène glycol, de butylène glycol, de glycérol, de tétraglycol, de 1,2-hexanediol, de 1,2-butanediol, de PEG 400 et de polyglycérol, de préférence de propylène glycol.

3. Composition de DES selon la revendication 1, dans laquelle le constituant DES comprend au moins un constituant de solvant eutectique profond d'origine naturelle (NADES).

4. Composition de DES selon l'une quelconque des revendications précédentes, dans laquelle le constituant DES est choisi dans le groupe constitué d'acides organiques, de composés phénoliques, de terpénoïdes, d'acides gras, de bases organiques, de sucres ou d'édulcorants, de glycols, d'acides aminés, de composés d'ammonium quaternaire tels que des composés de choline, et de dérivés de ces classes.

5. Composition de DES selon la revendication 1, comprenant un ou plusieurs parmi : un acide organique pouvant être un parmi l'acide malique, l'acide citrique, l'acide lactique, l'acide fumarique, l'acide tartrique, l'acide ascorbique, l'acide pimélique, l'acide gluconique et l'acide acétique et/ou des dérivés de ceux-ci tels que le nicotinamide ; un ou plusieurs composés phénoliques, pouvant être un parmi le propénylguéthol-isoeugénol, le tocophérol, le gallate de propyle, la tyramine, le butylparabène et la vanilline ; un ou plusieurs terpénoïdes, pouvant être un parmi le terpinéol, l'alcool périllylique et le menthol ; un ou plusieurs acides gras, pouvant être un parmi l'acide nonanique, l'acide octanoïque, l'acide laurique, le myristate d'isopropyle, le palmitate d'ascorbyle ; une ou plusieurs bases organiques, pouvant être une parmi l'urée et la guanine ; un sucre ou édulcorant pouvant être un parmi le saccharose, le glucose, le fructose ou le lactose ; un acide aminé qui peut être un parmi l'alanine, l'acide glutamique ou le glutamate ; des composés d'ammonium quaternaire pouvant être le chlorure de choline, le mononitrate de thiamine ou la carnitine.

6. Composition de DES selon l'une quelconque des revendications précédentes, dans laquelle le ou les constituant(s) de DES/NADES sont fournis à un rapport molaire dans la plage comprise entre 0,1 à 1 et 4 à 1 pour chaque constituant par rapport au constituant solubilisant polymère, de préférence dans la plage entre 0,2 à 1 et 1,75 à 1 et de manière plus préférée dans la plage comprise entre de 0,3 à 1 et 1,5 à 1.

7. Composition de DES selon l'une quelconque des revendications précédentes, comprenant en outre un ingrédient pharmaceutique actif (API).

8. Composition de DES selon l'une quelconque des revendications précédentes, comprenant en outre un ou plusieurs inhibiteurs de précipitation polymères (PPI).

9. Composition de DES selon la revendication 8, dans laquelle le rapport pondéral entre PPI(s) et API(s) est compris entre 0,2 à 1 et 20 à 1, de préférence entre 0,35 à 1 et 10 à 1, ou de manière plus préférée entre 0,5 à 1 et 5 à 1.

10. Composition de DES selon l'une quelconque des revendications précédentes, comprenant en outre un ou plusieurs délitants, de préférence présents à un pourcentage de masse compris entre 0,25 et 10 %, ramené à la masse du DES total.

11. Composition de DES selon l'une quelconque des revendications précédentes destinée à une administration orale.

12. Procédé de préparation d'une composition pharmaceutique, comprenant les étapes suivantes consistant à : mélanger un glycol et le solubilisant polymère tel que défini dans la revendication 1 pour créer un liquide ; ajouter un ou plusieurs constituants de DES, et de préférence des constituants de NADES audit liquide en formant un DES ; et solubiliser une ou plusieurs API(s) dans ledit DES.

13. Procédé selon la revendication 12, dans lequel un ou plusieurs PPI(s) sont solubilisés dans ladite composition pharmaceutique.

14. Procédé selon la revendication 12 ou la revendication 13, dans lequel un délitant est mélangé dans ladite composition pharmaceutique.

15. Utilisation d'une combinaison d'un glycol avec un composant solubilisant polymère dans la préparation d'une composition de solvant eutectique profond (DES), dans laquelle le composant solubilisant polymère est choisi dans le groupe constitué d'esters et de lactones d'acides organiques ; d'acides dicarboxyliques ; d'esters d'acides dicarboxyliques ; d'esters, d'éthers et de carbonates de diols et de triols ; et de mélanges de ceux-ci, de préférence dans laquelle le glycol et le composant solubilisant polymère sont utilisés dans un rapport molaire dans la plage entre de 12 à 1 et 1 à 10, de manière plus préférée dans la plage entre 8 à 1 et 1 à 1,5, et de la manière la plus préférée dans la plage entre 4 à 1 et 1 à 1.
